# DEMANDE DE BREVET EUROPEEN

(11) **EP 0 974 586 A1**
(43) Date de publication de la demande: **26.01.2000**
(21) Numéro de dépôt: 99401719.2
(22) Date de dépôt: 08.07.1999
(51) Int. Cl.: C07D 239/48, A61K 7/06, A61K 31/505

(54) **Nouveaux 2-amino, 4-alkylamino pyrimide 3-oxydes et composition les comprenant**

(30) Priorité: 24.07.1998 FR 9809509
(71) Demandeur: L'OREAL, 75008 Paris (FR)
(72) Inventeur: Mahe, Yann, 91390 MorsangS/Orge (FR); Michelet, Jean-François, 94000 Créteil (FR); Pichaud, Patrick, 78140 Velizy (FR); Galey, Jean-Baptiste, 93600 Aulnay (FR)
(74) Mandataire: Tezier Herman, Béatrice

(57) **Abrégé**

La présente invention concerne de nouveaux composés chimiques de la famille des 2-amino, 4-alkylamino pyrimidine 3-oxydes, les compositions les contenant ainsi que leur utilisation à titre de principe actif pour induire et/ou stimuler la croissance des cheveux et/ou freiner leur chute.

## Description

La présente invention concerne de nouveaux composés chimiques de la famille des 2-amino, 4-alkylamino pyrimidine 3-oxydes, les compositions les contenant ainsi que leur utilisation à titre de principe actif pour induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Cependant différentes causes peuvent entraîner une perte importante, temporaire ou définitive, des cheveux. L'alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux. Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Il reste, d'une manière générale, qu'il serait intéressant et utile de pouvoir disposer de composés actifs autres que ceux déjà connus, potentiellement plus actifs et/ou moins toxiques.

Ce but et d'autres sont atteints par la présente invention qui concerne de nouveaux composés de la famille des 2-amino, 4-alkylamino pyrimidine 3-oxydes répondant à la formule générale (I) : 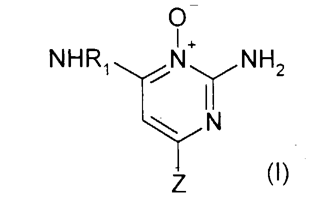 dans laquelle
R₁ représente un groupement alkyle ayant de 5 à 20 atomes de carbone,
et Z représente soit un atome d'hydrogène, soit le radical -OR₂, dans lequel R² représente un groupement alkyle ayant de 1 à 12 atomes de carbone,
ainsi que ses formes acylées ou ses sels d'addition d'acides.

Ces composés présentent des activités remarquables qui justifient leur utilisation comme principe actif pour induire et/ou stimuler la croissance des cheveux et/ou freiner leur chute.

On sait que certains acides gras polyinsaturés, en particulier ceux présentant 20 atomes de carbone, tels que l'acide arachidonique, l'acide dihomo γ-linolénique ou bien encore l'acide éicosapentaénoïque, peuvent être transformés in vivo, sous l'action de certaines enzymes spécifiques contenues dans les cellules vivantes, en particulier les cellules épithéliales, en certains autres composés de type éicosanoïdes utiles à l'organisme.

Ainsi, il est connu que les enzymes dites cyclo-oxygénases génèrent, à partir des différents acides gras mentionnés ci-dessus, particulièrement à partir de l'acide arachidonique, des éicosanoïdes de type prostaglandines et thromboxanes, et que les enzymes dites lipoxygénases sont, quant à elles, responsables de la formation des éicosanoïdes de type leukotriènes et autres acides acycliques hydroxylés à 20 atomes de carbone. Un même acide gras polyinsaturé donné (ou substrat) pourra donner lieu, selon la nature de l'enzyme avec laquelle il aura réagi en premier, à la formation de plusieurs métabolites différents, à savoir par exemple des prostaglandines et des leukotriènes.

L'activité cyclooxygénase peut se définir comme l'activité enzymatique qui transforme certains acides gras polyinsaturés en produits oxygénés cyclisés qui sont en fait des endoperoxydes hautement instables qui entrent par la suite dans des voies métaboliques subséquentes.
La prostaglandine-endoperoxyde synthase, ou cyclooxygénase (ou PGHS, EC 1.14.99.1) qui est une hémoprotéine est un exemple de ces enzymes présentant une telle activité. Elle intervient dans l'une des voies métaboliques des prostaglandines.

On sait que les transformations enzymatiques ci-dessus exposées et les différents produits de réaction qui en résultent ont une influence non négligeable sur les mécanismes de pousse du poil et/ou du cheveu.
A ce titre la demanderesse a montré qu'en privilégiant l'une ou l'autre des deux voies enzymatiques, cyclo-oxygénases ou lipoxygénases, au sein des cellules de la peau et/ou du cuir chevelu, on pouvait modifier de manière substantielle la pousse des poils et/ou des cheveux. Ceci a fait l'objet de la demande de brevet EP 94-402055.
Essentiellement, dans cette demande de brevet, la demanderesse préconisait de favoriser l'une des voies par rapport à l'autre par l'administration de combinaison de composés associant un inhibiteur de l'une des voies à un stimulateur de l'autre voie.

Plus précisément encore, la demanderesse a montré que l'on peut favoriser la pousse des poils et/ou des cheveux et/ou lutter contre leur chute en privilégiant la voie des cyclo-oxygénases, par exemple par l'activation de la PGHS et l'inhibition de la voie des lipoxygénases.

L'implication de ces enzymes dans de nombreuses voies métaboliques et ainsi les conséquences que pourrait avoir un dérèglement de leur fonctionnement font que de nombreuses recherches ont été entreprises afin de mettre en évidence des substances ayant la capacité soit d'augmenter soit de diminuer l'activité de ces enzymes.
Dans le domaine des activateurs de cyclo-oxygénase, on connaît notamment les métabolites de l'acide arachidonique, le monoxyde d'azote et les composés donneurs de monoxyde d'azote, le stanozolol, les composés donneurs de glutathion, les ionophores calciques, les anthocyanosides, les bioflavonoïdes, les facteurs activateurs de plaquettes (PAF), les cytokines pro-inflammatoires, les endotoxines bactériennes.
De même, on peut citer la 6-chloro-2,3-dihydroxy-1,4-naphtoquinone (CNDQ), qui a la particularité d'être à la fois un inhibiteur des lipoxygénases et un stimulateur des cyclo-oxygénases (C.J. Bedord et al., The Journal of Investigative Dermatology, 81:566-571, 1983).
Cependant, la plupart de ces substances présente comme inconvénient majeur d'être à large spectre fonctionnel, ce qui entraîne qu'elles ne présentent pas en général de véritable spécificité pour la cyclooxygénase. A cet égard la littérature traitant de ce sujet montre une grande variété d'interprétation. Ces substances peuvent être également labiles ou leur activité dépendante de leur concentration, ce qui rend leur utilisation délicate.

La demanderesse a donc cherché de nouveaux composés ayant une activité au moins sur la PGHS, composés qui entreraient alors dans la catégorie des activateurs de cyclooxygénase.

De manière surprenante et inattendue après de longs travaux, la demanderesse a découvert que les 2-amino, 4-alkylamino pyrimidine 3-oxydes de l'invention présentent la propriété d'activer la PGHS.

De plus, la présence d'une chaîne alkyle en position 4 confère à ces composés des propriétés lipophiles améliorées.
Ceci renforce l'intérêt de ces composés comme actifs dans le traitement de la chute des cheveux.

Ainsi, l'invention a pour objet un composé répondant à la formule générale (I) : 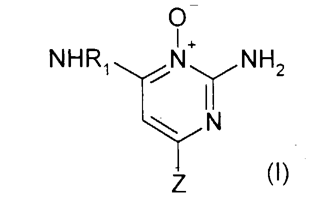 dans laquelle
R₁ représente un groupement alkyle ayant de 5 à 20 atomes de carbone,
et Z représente soit un atome d'hydrogène, soit le radical -OR₂, dans lequel R₂ représente un groupement alkyle ayant de 1 à 12 atomes de carbone,
ainsi que ses formes acylées ou ses sels d'addition d'acides.

Par radical alkyle, on entend selon l'invention un radical acyclique, linéaire ou ramifié, provenant de l'enlèvement d'un atome d'hydrogène dans la molécule d'un hydrocarbure, comme par exemple un radical méthyle, éthyle, propyle, butyle, pentyle, hexyle, heptyle, octyle, nonyle, décyle, undécyle, dodécyle, tridécyle, tétradécyle, pentadécyle, hexadécyle, heptadécyle, octodécyle, nonadécyle, eicosadécyle.

Selon une forme de réalisation préférentielle de l'invention, R₁ peut être un groupement alkyle ayant de 6 à 12 atomes de carbone, comme par exemple un radical hexyle, heptyle, octyle, nonyle, décyle, undécyle, dodécyle.

Selon une autre forme de réalisation préférentielle de l'invention, R₂ peut être un groupement alkyle ayant de 2 à 6 atomes de carbone comme par exemple un radical éthyle, propyle, butyle, pentyle, hexyle.

Préférentiellement, le composé de l'invention est choisi parmi le
2-amino, 4-pentylamino pyrimidine 3-oxyde,
2-amino, 4-hexylamino pyrimidine 3-oxyde,
2-amino, 4-heptylamino pyrimidine 3-oxyde,
2-amino, 4-octylamino pyrimidine 3-oxyde,
2-amino, 4-nonylamino pyrimidine 3-oxyde,
2-amino, 4-décylamino pyrimidine 3-oxyde,
2-amino, 4-undécylamino pyrimidine 3-oxyde,
2-amino, 4-dodécylamino pyrimidine 3-oxyde,
2-amino, 4-tridécylamino pyrimidine 3-oxyde,
2-amino, 4-tétradécylamino pyrimidine 3-oxyde,
2-amino, 4-pentadécylamino pyrimidine 3-oxyde,
2-amino, 4-hexadécylamino pyrimidine 3-oxyde,
2-amino, 4-heptadécylamino pyrimidine 3-oxyde,
2-amino, 4-octatadécylamino pyrimidine 3-oxyde,
2-amino, 4-nonadécylamino pyrimidine 3-oxyde,
2-amino, 4-eicosadécylamino pyrimidine 3-oxyde,
2-amino, 4-pentylamino, 6-méthoxy pyrimidine 3-oxyde,
2-amino, 4-hexylamino, 6-méthoxy pyrimidine 3-oxyde,
2-amino, 4-heptylamino, 6-méthoxy pyrimidine 3-oxyde,
2-amino, 4-octylamino, 6-méthoxy pyrimidine 3-oxyde,
2-amino, 4-nonylamino, 6-méthoxy pyrimidine 3-oxyde,
2-amino, 4-décylamino, 6-méthoxy pyrimidine 3-oxyde,
2-amino, 4-undécylamino, 6-méthoxy pyrimidine 3-oxyde,
2-amino, 4-dodécylamino, 6-méthoxy pyrimidine 3-oxyde,
2-amino, 4-tridécylamino, 6-méthoxy pyrimidine 3-oxyde,
2-amino, 4-tétradécylamino, 6-méthoxy pyrimidine 3-oxyde,
2-amino, 4-pentadécylamino, 6-méthoxy pyrimidine 3-oxyde,
2-amino, 4-hexadécylamino, 6-méthoxy pyrimidine 3-oxyde,
2-amino, 4-heptadécylamino, 6-méthoxy pyrimidine 3-oxyde,
2-amino, 4-octatadécylamino, 6-méthoxy pyrimidine 3-oxyde,
2-amino, 4-nonadécylamino, 6-méthoxy pyrimidine 3-oxyde,
2-amino, 4-eicosadécylamino, 6-méthoxy pyrimidine 3-oxyde,
2-amino, 4-pentylamino, 6-éthoxy pyrimidine 3-oxyde,
2-amino, 4-hexylamino, 6-éthoxy pyrimidine 3-oxyde,
2-amino, 4-heptylamino, 6-éthoxy pyrimidine 3-oxyde,
2-amino, 4-octylamino, 6-éthoxy pyrimidine 3-oxyde,
2-amino, 4-nonylamino, 6-éthoxy pyrimidine 3-oxyde,
2-amino, 4-décylamino, 6-éthoxy pyrimidine 3-oxyde,
2-amino, 4-undécylamino, 6-éthoxy pyrimidine 3-oxyde,
2-amino, 4-dodécylamino, 6-éthoxy pyrimidine 3-oxyde,
2-amino, 4-tridécylamino, 6-éthoxy pyrimidine 3-oxyde,
2-amino, 4-tétradécylamino, 6-éthoxy pyrimidine 3-oxyde,
2-amino, 4-pentadécylamino, 6-éthoxy pyrimidine 3-oxyde,
2-amino, 4-hexadécylamino, 6-éthoxy pyrimidine 3-oxyde,
2-amino, 4-heptadécylamino, 6-éthoxy pyrimidine 3-oxyde,
2-amino, 4-octatadécylamino, 6-éthoxy pyrimidine 3-oxyde,
2-amino, 4-nonadécylamino, 6-éthoxy pyrimidine 3-oxyde,
2-amino, 4-eicosadécylamino, 6-éthoxy pyrimidine 3-oxyde,
2-amino, 4-pentylamino, 6-propyloxy pyrimidine 3-oxyde,
2-amino, 4-hexylamino, 6-propyloxy pyrimidine 3-oxyde,
2-amino, 4-heptylamino, 6-propyloxy pyrimidine 3-oxyde,
2-amino, 4-octylamino, 6-propyloxy pyrimidine 3-oxyde,
2-amino, 4-nonylamino, 6-propyloxy pyrimidine 3-oxyde,
2-amino, 4-décylamino, 6-propyloxy pyrimidine 3-oxyde,
2-amino, 4-undécylamino, 6-propyloxy pyrimidine 3-oxyde,
2-amino, 4-dodécylamino, 6-propyloxy pyrimidine 3-oxyde,
2-amino, 4-tridécylamino, 6-propyloxy pyrimidine 3-oxyde,
2-amino, 4-tétradécylamino, 6-propyloxy pyrimidine 3-oxyde,
2-amino, 4-pentadécylamino, 6-propyloxy pyrimidine 3-oxyde,
2-amino, 4-hexadécylamino, 6-propyloxy pyrimidine 3-oxyde,
2-amino, 4-heptadécylamino, 6-propyloxy pyrimidine 3-oxyde,
2-amino, 4-octatadécylamino, 6-propyloxy pyrimidine 3-oxyde,
2-amino, 4-nonadécylamino, 6-propyloxy pyrimidine 3-oxyde,
2-amino, 4-eicosadécylamino, 6-propyloxy pyrimidine 3-oxyde,
2-amino, 4-pentylamino, 6-butyloxy pyrimidine 3-oxyde,
2-amino, 4-hexylamino, 6-butyloxy pyrimidine 3-oxyde,
2-amino, 4-heptylamino, 6-butyloxy pyrimidine 3-oxyde,
2-amino, 4-octylamino, 6-butyloxy pyrimidine 3-oxyde,
2-amino, 4-nonylamino, 6-butyloxy pyrimidine 3-oxyde,
2-amino, 4-décylamino, 6-butyloxy pyrimidine 3-oxyde,
2-amino, 4-undécylamino, 6-butyloxy pyrimidine 3-oxyde,
2-amino, 4-dodécylamino, 6-butyloxy pyrimidine 3-oxyde,
2-amino, 4-tridécylamino, 6-butyloxy pyrimidine 3-oxyde,
2-amino, 4-tétradécylamino, 6-butyloxy pyrimidine 3-oxyde,
2-amino, 4-pentadécylamino, 6-butyloxy pyrimidine 3-oxyde,
2-amino, 4-hexadécylamino, 6-butyloxy pyrimidine 3-oxyde,
2-amino, 4-heptadécylamino, 6-butyloxy pyrimidine 3-oxyde,
2-amino, 4-octatadécylamino, 6-butyloxy pyrimidine 3-oxyde,
2-amino, 4-nonadécylamino, 6-butyloxy pyrimidine 3-oxyde,
2-amino, 4-eicosadécylamino, 6-butyloxy pyrimidine 3-oxyde,
et leurs isomères ramifiés .

Préférentiellement selon l'invention on choisit le composé parmi le :
2-amino, 4-hexylamino pyrimidine 3-oxyde,
2-amino, 4-octylamino pyrimidine 3-oxyde,
2-amino, 4-dodécylamino pyrimidine 3-oxyde,
2-amino, 4-octylamino, 6-butyloxy pyrimidine 3-oxyde,
et encore plus préférentiellement parmi le
2-amino, 4-dodécylamino pyrimidine 3-oxyde,
2-amino, 4-octylamino, 6-butyloxy pyrimidine 3-oxyde.

Un deuxième objet de l'invention est un procédé de préparation des 2-amino, 4-alkylamino pyrimidine 3-oxyde de formule (I) tels que définis ci-dessus.

Ce procédé est caractérisé par le fait que l'on fait réagir dans un solvant comme l'éthanol une amine aliphatique et de la 2-amino, 4,6-dichloropyrimidine. Le composé ainsi obtenu après purification est ensuite mis à réagir, dans un solvant comme l'isopropanol avec un complexe Urée/H₂O₂ et de l'anhydride phtalique. Après purification, le composé obtenu est mis à réagir dans un solvant comme l'éthanol absolu en présence de potasse et de palladium sur charbon sous pression d'hydrogène pour obtenir les 2-amino, 4-alkylamino pyrimidine 3-oxyde correspondant.

Un troisième objet de l'invention est un procédé de préparation des 2-amino, 4-alkylamino, 6-alkyloxy pyrimidine 3-oxyde de formule (I) tels que définis ci-dessus.

Ce procédé est caractérisé par le fait que l'on fait réagir dans un solvant comme l'éthanol une amine aliphatique et de la 2-amino, 4,6-dichloropyrimidine. Le composé ainsi obtenu après purification est ensuite mis à réagir dans un solvant comme l'isopropanol avec un complexe Urée/H₂O₂ et de l'anhydride phtalique. Après purification, le composé obtenu est mis à réagir avec un excès d'alkanolate de sodium ou potassium pour obtenir le 2-amino, 4-alkylamino, 6-alkyloxy pyrimidine 3-oxyde correspondant.

Des exemples de préparation des composés selon l'invention sont donnés par ailleurs dans les exemples.

Un quatrième objet de l'invention concerne des compositions qui comprennent au moins un composé de la famille des 2-amino, 4-alkylamino pyrimidine 3-oxydes répondant à la formule générale (I) tel défini ci-dessus.

Bien entendu les compositions selon l'invention peuvent comprendre les composés de formule (I) seuls ou en mélanges en toutes proportions.

La quantité efficace de composé à utiliser correspond bien entendu à la quantité nécessaire pour obtenir le résultat désiré. L'homme du métier est donc en mesure d'évaluer cette quantité efficace qui dépend de la nature du composé utilisé et de la personne ainsi traitée.
Pour donner un ordre de grandeur, dans la composition selon l'invention, le composé peut être présent à une concentration comprise entre 0.001 % et 10 %, en poids par rapport au poids total de la composition et de préférence comprise entre 0.01 % et 2 %.

Un cinquième objet de l'invention concerne l'utilisation à titre de principe actif, dans un milieu physiologiquement acceptable, dans une composition d'une quantité efficace d'au moins un composé de formule (I), ce composé ou les compositions étant destinés à induire et/ou stimuler la croissance des cheveux et/ou freiner leur chute.

La composition selon l'invention peut être ingérée, injectée ou appliquée sur la peau et/ou les cheveux. Selon le mode d'administration, la composition selon l'invention peut se présenter sous toutes les formes galéniques normalement utilisées.

Pour une application topique sur la peau ou les cheveux, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats ou dérivés de protéines (dérivés d'α-, les acides aminés, les olyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés. Selon l'invention la composition peut associer au moins composé de formule (I) à d'autres agents actifs. Parmi ces agents actifs, on peut citer à titre d'exemple :
- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélateurs de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale, marine ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoïdes, les acides eicosatétraénoïque et eicosatriénoïque ou leurs esters et amides

Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale, marine ou bactérienne.

On peut également envisager que la composition comprenant au moins un composé tel que défini précédemment soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition selon l'invention est à appliquer sur les zones alopéciques du cuir chevelu et des cheveux d'un individu, et est éventuellement laissée en contact plusieurs heures et est éventuellement à rincer. On peut, par exemple, appliquer la composition contenant une quantité efficace d'au moins un composé tel que défini précédemment, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, caractérisé par le fait qu'il consiste à appliquer sur les cheveux et/ou le cuir chevelu, une composition comprenant une quantité efficace d'au moins un composé tel que défini précédemment, à laisser celle-ci en contact avec les cheveux et/ou le cuir chevelu, et éventuellement à rincer.

Le procédé de traitement présente les caractéristiques d'un procédé cosmétique dans la mesure où il permet d'améliorer l'esthétique des cheveux en leur donnant une plus grande vigueur et un aspect amélioré.

La composition selon l'invention peut être destinée à un usage cosmétique ou pharmaceutique, particulièrement dermatologique.

On va maintenant donner à titre d'illustration des exemples qui ne sauraient limiter en aucune façon la portée de l'invention.

### Exemple 1 : synthèse du 2-amino, 4-hexylaminopyrimidine 3-oxyde :

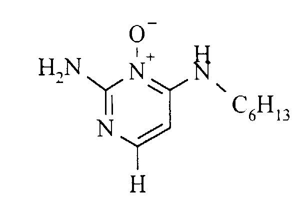

1) synthèse de la 2-amino, 4-hexylamino, 6-chloro pyrimidine : 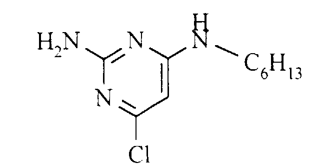
   Dans un réacteur, on met en suspension 50 g de 2-amino-4,6-dichloropyrimidine dans 350 ml d'éthanol absolu. On ajoute en une seule fois 181,5 ml d'hexylamine (réactif A) et on porte au reflux pendant 3h. On évapore milieu sous vide. L'huile obtenue est reprise dans 600 ml d'eau sous agitation pendant 1h30. Le précipité est filtré, lavé, séché au dessiccateur chauffant. On obtient ainsi 43,5 g pour un rendement de 62 %.
   Analyses
   * Spectre RMN ¹H (200MHz; DMSO) δ ppm: 0.8 (3H, t), 1.2 (6H, s), 1.4 (2H, t), 3(2H, s), 5.6 (1H, s), 6.2 (2H, s), 6.9 (1H, s).
2) synthèse de la 2-amino, 4-hexylamino, 6-chloropyrimidine-3-oxyde : 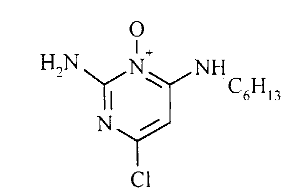
   Dans un tricol, on met en suspension 5,95 g de complexe urée / H₂O₂ et 9,07 g d'anhydride phtalique dans 90 ml d'isopropanol. On laisse agiter 30' à température ambiante (20-25 °C). On ajoute ensuite 10 g de 2-amino, 4-hexylamino, 6-chloro pyrimidine tout en controlant l'exotherme à +30°C. Après 3 heures de réaction, on verse 100 ml d'hydrogénosulfite de sodium pour détruire les oxydants résiduels. On laisse décanter et on concentrer la phase supérieure sous vide. On reprend du résidu obtenu dans un mélange eau (80 ml)/ soude à 30% (20 ml).Le solide obtenu est réempaté dans 150 ml d'éther isopropylique. On filtre et on lave le solide. Ce dernier est séché au dessiccateur chauffant. On obtient ainsi 4,52 g pour un rendement de 42 %.
   Analyses
   * Spectre RMN ¹H (200MHz; DMSO) δ ppm: 0.7 (3H, t), 1 (6H, s), 1.2 (2H, t), 3.1(2H, m),6 (1H, s), 7.4 (2H, m), 7.7 (1H, t).
3) synthèse de la 2-amino, 4-hexylamino pyrimidine-3-oxyde 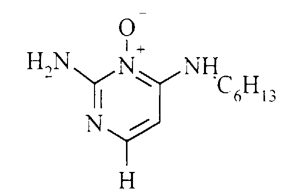 Dans un tricol, on dissout 0,7 g de potasse dans 100 ml d'éthanol absolu. On ajoute ensuite 2,2 g de 2-amino, 4-hexylamino, 6-chloropyrimidine 3-oxyde. Après solubilisation complète, on ajoute 0,5 g de palladium sur charbon et on met en réaction dans un hydrogénateur sous 3 bars de pression d'hydrogène. Après 2 heures on filtre le milieu sur papier filtre et on concentre sous vide. Le solide jaune pâle obtenu est recristallisé dans 20 ml d'acétonitrile. On filtre et on lave le gâteau par 10 ml d'eau. On sèche le solide au dessiccateur sous vide. On obtient ainsi 1 g de composé pour un rendement de 53%.
   Analyses
   * Spectre RMN ¹H (200MHz; DMSO) δ ppm: 1.9 (3H, t), 2.2 (6H, s), 2.5 (2H, t), 4.3 (2H, m), 7.2 (1H, d), 8.1 (2H, s), 8.5 (1H, d), 8.6 (1H,m).

### Exemple 2 : synthèse du 2-amino, 4-dodécylamino pyrimidine 3-oxyde :

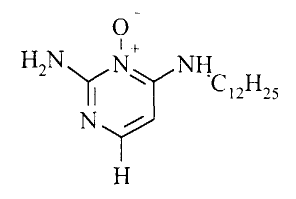

1) synthèse de la 2-amino, 4-dodécylamino, 6-chloro pyrimidine : 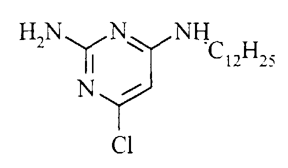
   Dans un réacteur, on met en suspension 10 g de 2-amino-4,6-dichloropyrimidine dans 100 ml d'éthanol absolu. On ajoute en une seule fois 33,9 g de dodécylamine (réactif A) et on porte au reflux pendant 2 h. On évapore le milieu sous vide. Le solide obtenu est repris dans 200 ml d'acétone sous agitation pendant 30'. Ajouter 200ml d'eau et laisser agiter 2 h. Le solide est filtré, lavé, séché au dessiccateur chauffant.
   On obtient ainsi 17,55 g pour un rendement de 92 %.
   Analyses
   * Spectre RMN ¹H (200MHz; DMSO) δ ppm: 0.8 (3H, t), 1.2 (18H, s), 1.3 (2H, t), 3(2H, s), 5.6 (1H, s), 6.1 (2H, s), 6.8 (1H, s).
2) synthèse de la 2-amino, 4-dodécylamino, 6-chloropyrimidine 3-oxyde : 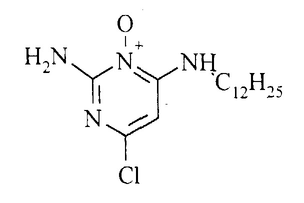
   Dans un tricol, on met en suspension 3,26 g de complexe urée / H₂O₂ et 4,97 g d'anhydride phtalique dans 60 ml d'isopropanol. On laisse agiter 30' à l'ambiante. On ajoute ensuite 10 g de 2-amino, 4-dodécylamino, 6-chloro pyrimidine tout en controlant l'exotherme à +25°C. Après 2 h de réaction, on refoidit le milieu à +4°C pendant 1h. On filtre le solide et on le lave par 5 ml de méthanol glacé. On reprend le solide dans un mélange eau (12 ml)/ soude à 40% (3 ml). On filtre et on lave le solide. Ce dernier est séché au dessiccateur chauffant.
   On obtient ainsi 3,3 g pour un rendement de 42 %.
   Analyses
   * Spectre RMN ¹H (200MHz; DMSO) δ ppm: 0.7 (3H, t), 1.1 (18H, s), 1.3 (2H, t), 2.8(2H, t), 5.5 (1H, s), 6.9 (3H, m).
3) synthèse du 2-amino, 4-dodécylamino pyrimidine 3-oxyde : 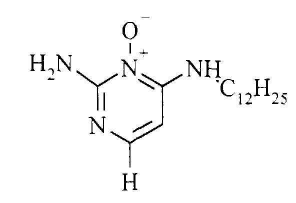
   Dans un tricol, on dissout 0,7 g de potasse dans 250 ml d'éthanol absolu. On ajoute ensuite 3,3 g de 2-amino, 4-dodécylamino, 6-chloropyrimidine 3-oxyde. On attend la solubilsation complète. On ajoute 0,5 g de palladium sur charbon et on met en réaction dans un hydrogénateur sous 3 bars de pression d'hydrogène. Après 2h, on filtre le milieu sur papier filtre et on concentre sous vide. Le solide obtenu est recristallisé dans un mélange éthanol (10ml)/ acétonitrile (50ml). On filtre et on lave le gâteau par 10 ml d'eau. On sèche le solide au dessiccateur sous vide.
   On obtient ainsi 0,8 g pour un rendement de 27 %.
   Analyses
   * Spectre RMN ¹H (200MHz; DMSO) δ ppm: 0.9 (3H, t), 1.3 (18H, s), 1.7 (2H, t), 3.3 (2H, m), 6.3 (1H, d), 7.2 (2H, s), 7.6 (1H, d), 7.7 (1H, m).

### Exemple 3 : synthèse du 2-amino, 4-octylamino pyrimidine 3-oxyde

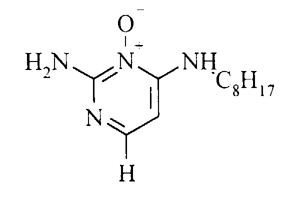

1) synthèse de la 2-amino,4-octylamino, 6-chloro pyrimidine 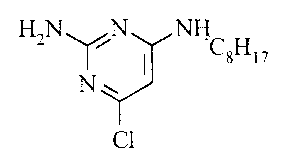
   Dans un réacteur, on met en suspension 20 g de 2-amino-4,6-dichloropyrimidine dans 200 ml d'éthanol absolu. On ajoute en une seule fois 60 g d'octylamine (réactif A) et on porte au reflux pendant 2 h. On évapore le milieu sous vide. L'huile obtenue est extraite par du dichlorométhane, puis est purifiée sur colonne de silice.
   On obtient ainsi 22,8 g pour un rendement de 71 %.
   Analyses
   RMN ¹H (200MHz; CDCl₃) δ ppm: 1 (3H, t), 1.4 (10H, s), 1.7 (2H, t), 3.3 (2H, m), 5.1 (3H, d), 6 (1H, s).
2) synthèse du 2-amino,4-octylamino, 6-chloro pyrimidine 3-oxyde 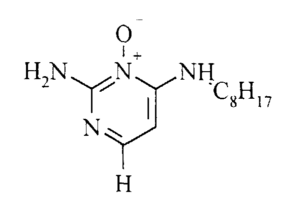
   Dans un tricol, on met en suspension 6,8 g de complexe urée / H₂O₂ et 10,35 g d'anhydride phtalique dans 100 ml d'isopropanol. On laisse agiter 30' à l'ambiante. On ajoute ensuite 10 g de 2-amino,4-octylamino, 6-chloro pyrimidine tout en contrôlant l'exotherme à +30°C. Après 3 heures de réaction, on verse 100 ml d'hydrogénosulfite de sodium pour détruire les oxydants résiduels. On laisse décanter et on concentre la phase supérieure sous vide. On reprend le résidu obtenu dans un mélange eau (80 ml)/ soude à 30 % (20 ml).On filtre et on lave le solide. Séchage de ce dernier au dessiccateur chauffant.
   On obtient ainsi 6,3 g pour un rendement de 46 %.
   Analyses
   RMN ¹H (200MHz; DMSO) δ ppm: 0.7 (3H, t), 1.1 (10H, s), 1.4 (2H, t), 3 (2H, t), 6 (1H, s), 7.2 (3H, s).
3) synthèse du 2-amino, 4-octylamino pyrimidine 3-oxyde 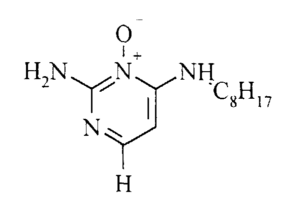
   Dans un tricol, on dissout 0,54 g de potasse dans 100ml d'éthanol absolu. On ajoute ensuite 2 g de 2-amino,4-octylamino, 6-chloro pyrimidine 3-oxyde. On attend la solubilisation complète. On ajoute 0,5 g de palladium sur charbon et on met en réaction dans un hydrogénateur sous 3 bars de pression d'hydrogène. Après 2 heures on filtre le milieu sur papier filtre et on concentre sous vide. Le solide jaune pâle obtenu est recristallisé dans 20 ml d'acétonitrile. On filtre et on lave le gâteau par 10 ml d'eau. Séchage du solide au dessiccateur sous vide.
   On obtient ainsi 0,5 g pour un rendement de 29 %.
   Analyses
   RMN ¹H (200MHz; DMSO) δ ppm: 1.1 (3H, t), 1.4 (10H, s), 1.8 (2H, t), 3.4 (2H, m), 6.1 (1H, d), 6.4 (2H, s), 7.3 (1H, t),7.7 (1H,d).

### Exemple 4-16 :

Selon le même protocole et en utilisant dans la première étape du procédé le réactif A approprié (voir tableau ci-dessous), il est possible d'obtenir les composés de l'invention listés ci-après :

| | Composé obtenu | Réactif A utilisé |
|---|---|---|
| 4 | 2-amino, 4-pentylamino pyrimidine 3-oxyde | pentylamine |
| 5 | 2-amino, 4-heptylamino pyrimidine 3-oxyde | heptylamine |
| 6 | 2-amino, 4-nonylamino pyrimidine 3-oxyde | nonylamine |
| 7 | 2-amino, 4-décylamino pyrimidine 3-oxyde | décylamine |
| 8 | 2-amino, 4-undécylamino pyrimidine 3-oxyde | undécylamine |
| 9 | 2-amino, 4-tridécylamino pyrimidine 3-oxyde | tridécylamine |
| 10 | 2-amino, 4-tétradécylamino pyrimidine 3-oxyde | tétradécylamine |
| 11 | 2-amino, 4-pentadécylamino pyrimidine 3-oxyde | pentadécylamine |
| 12 | 2-amino, 4-hexadécylamino pyrimidine 3-oxyde | hexadécylamine |
| 13 | 2-amino, 4-heptadécylamino pyrimidine 3-oxyde | heptadécylamine |
| 14 | 2-amino, 4-octatadécylamino pyrimidine 3-oxyde | octadécylamine |
| 15 | 2-amino, 4-nonadécylamino pyrimidine 3-oxyde | nonadécylamine |
| 16 | 2-amino, 4-eicosadécylamino pyrimidine 3-oxyde | eicosadécylamine |

### Exemple 17 : Synthèse du 2-amino, 4-octylamino, 6-butyloxy pyrimidine 3-oxyde :

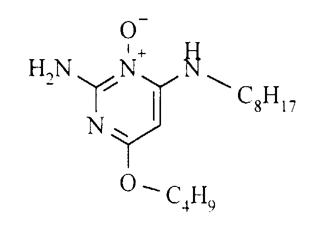

Dans un tricol de 250 ml, on introduit 90 ml de butanol (Réactif B) préalablement séché sur K₂CO₃. Sous argon on ajoute par petits morceaux, 0,18 g de sodium dans du kérosène. On chauffe ensuite le milieu à 60°C, jusqu'à solubilisation complète du sodium.

On introduit ensuite 1,5 g de 2-amino, 4-octylamino, 6-chloropyrimidine 3-oxyde obtenu selon l'exemple n°3 (réactif C), et on chauffe à 90°C pendant 24 heures.
On lave le milieu par une solution aqueuse à 20% en chlorure de sodium, puis on concentre la phase butanolique au rotavapor. Le résidu obtenu est purifié sur une colonne de silice. Le produit huileux obtenu en sortie de colonne est solubilisé dans 2,2 ml (2,5 eq) d'une solution méthanol chlorhydrique 2N, puis on évapore la solution sous pression réduite. On obtient un solide de couleur blanc cassé que l'on lave par 10 ml d'éther isopropylique. On obtient 0,5 g d'un solide blanc avec un rendement de 30 %.
Analyses :
RMN ¹H (200MHz; DMSO) δ ppm: 1 (6H;m), 1.7 (2H;s), 6.1 (16H;m), 3.4 (2H;q), 4.3 (2H;t), 5.7 (1H;s), 8.5 (4H;m)

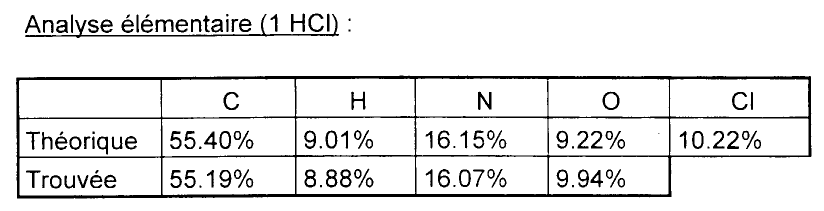

### Exemple 18 à 80 :

Selon le même protocole qu'à l'exemple 17, et en utilisant dans la première étape du procédé le réactif A approprié (voir tableau II ci-dessous), et dans la 2^{ème} étape les réactifs B et C appropriés (voir tableau II ci-dessous), il est possible d'obtenir les composés de l'invention listés ci-après (Tableau I) :

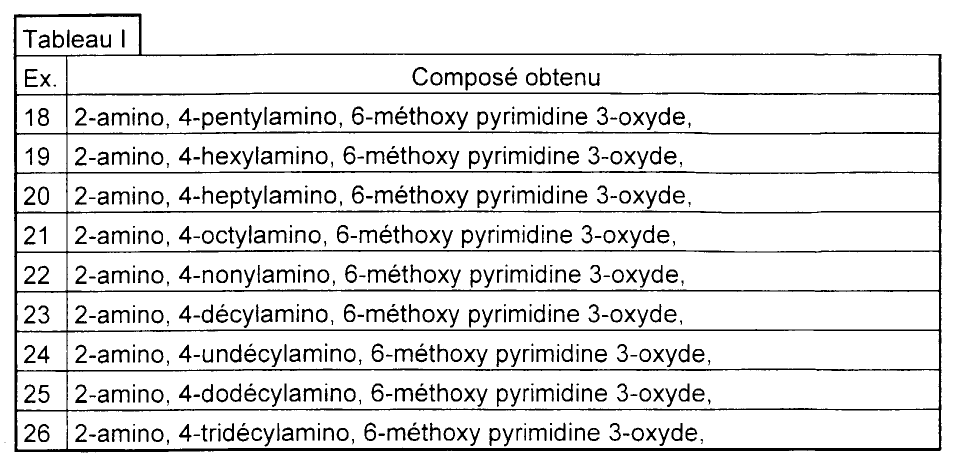

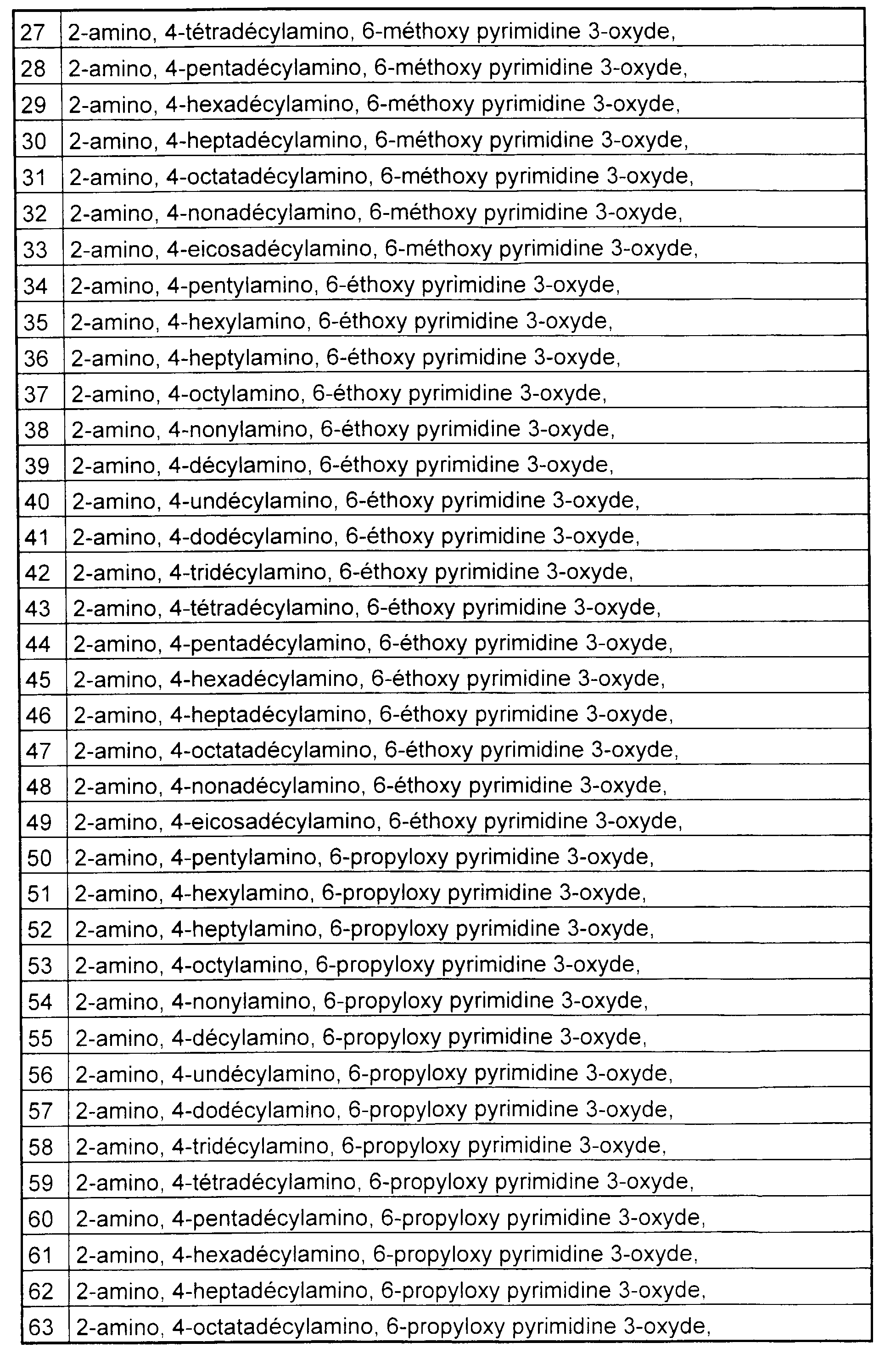

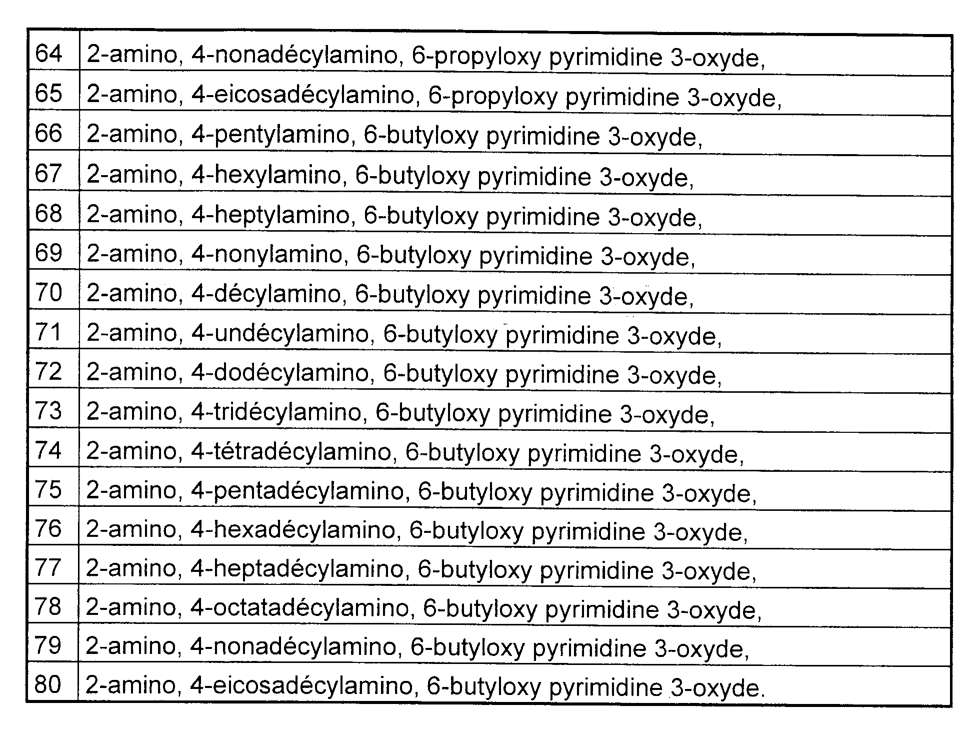

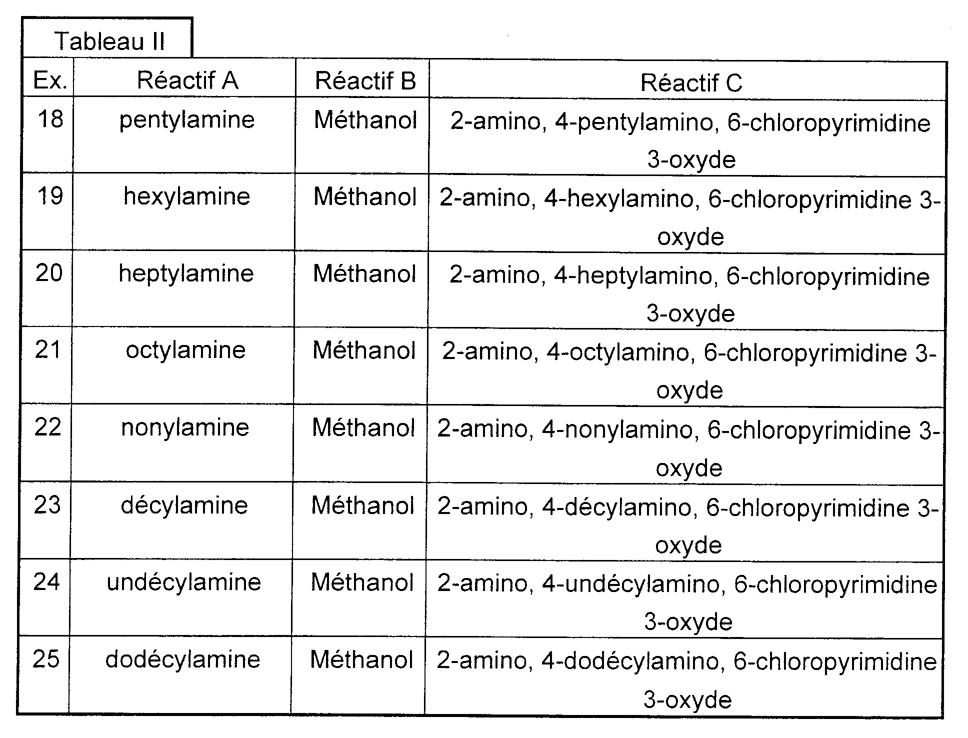

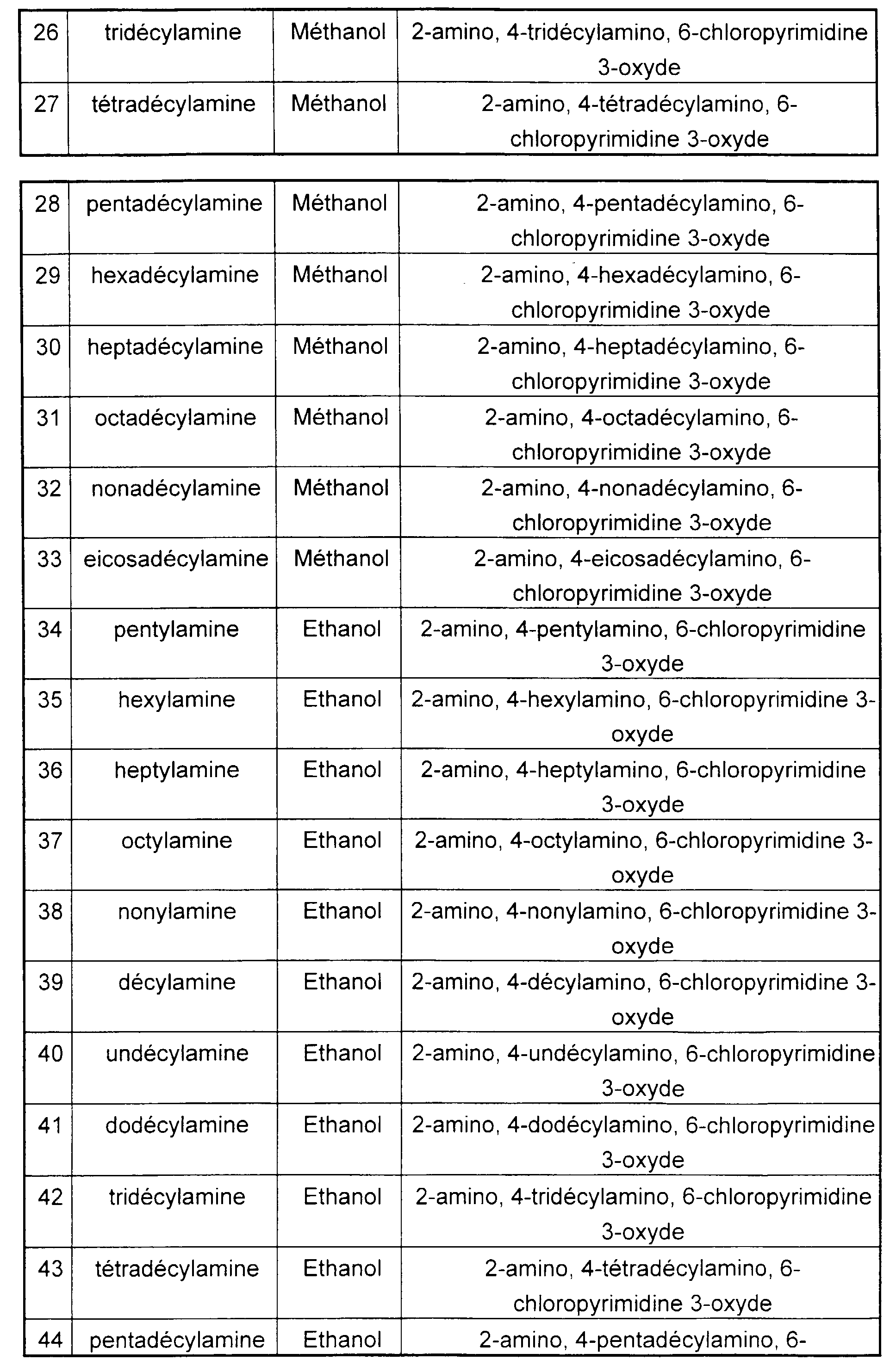

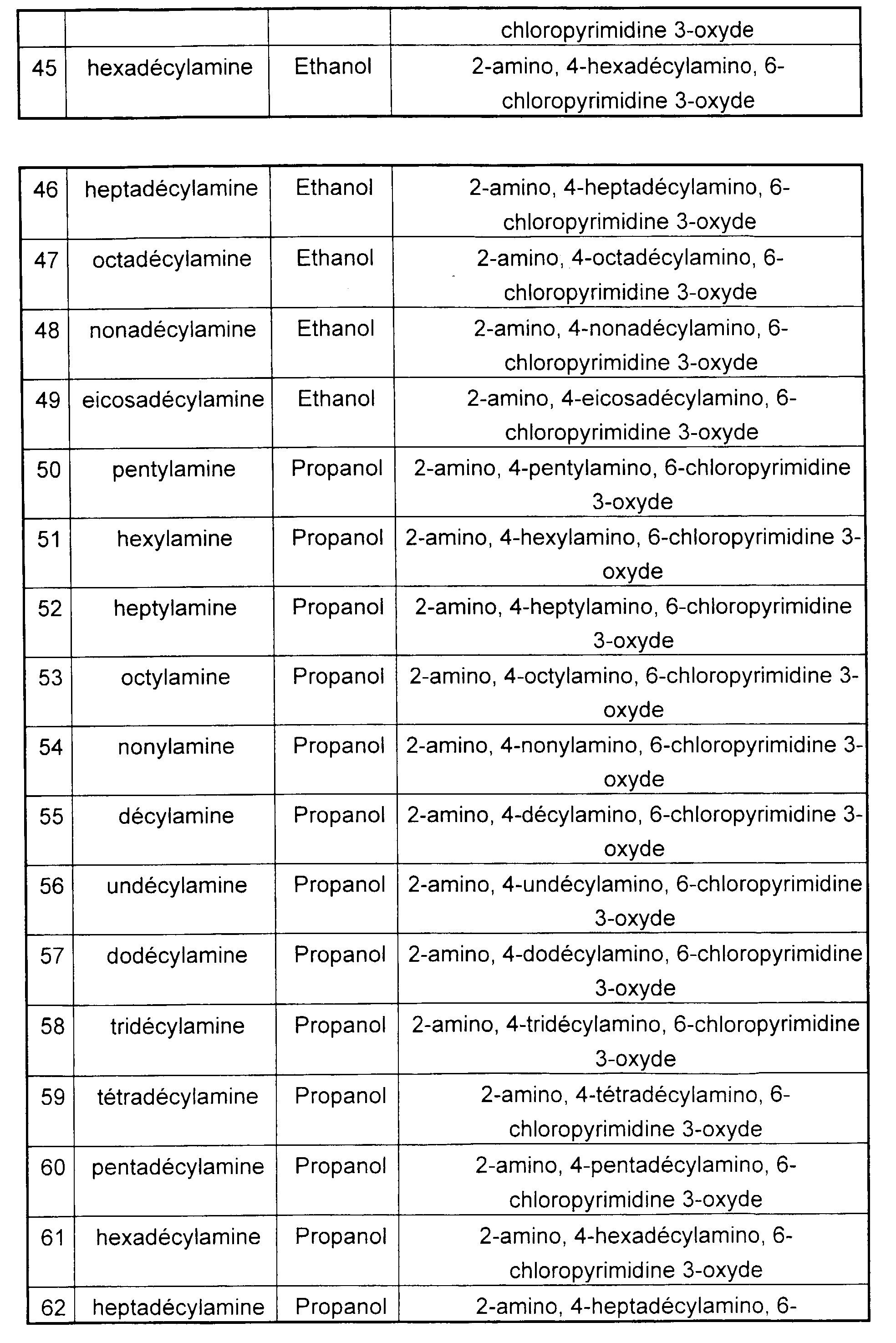

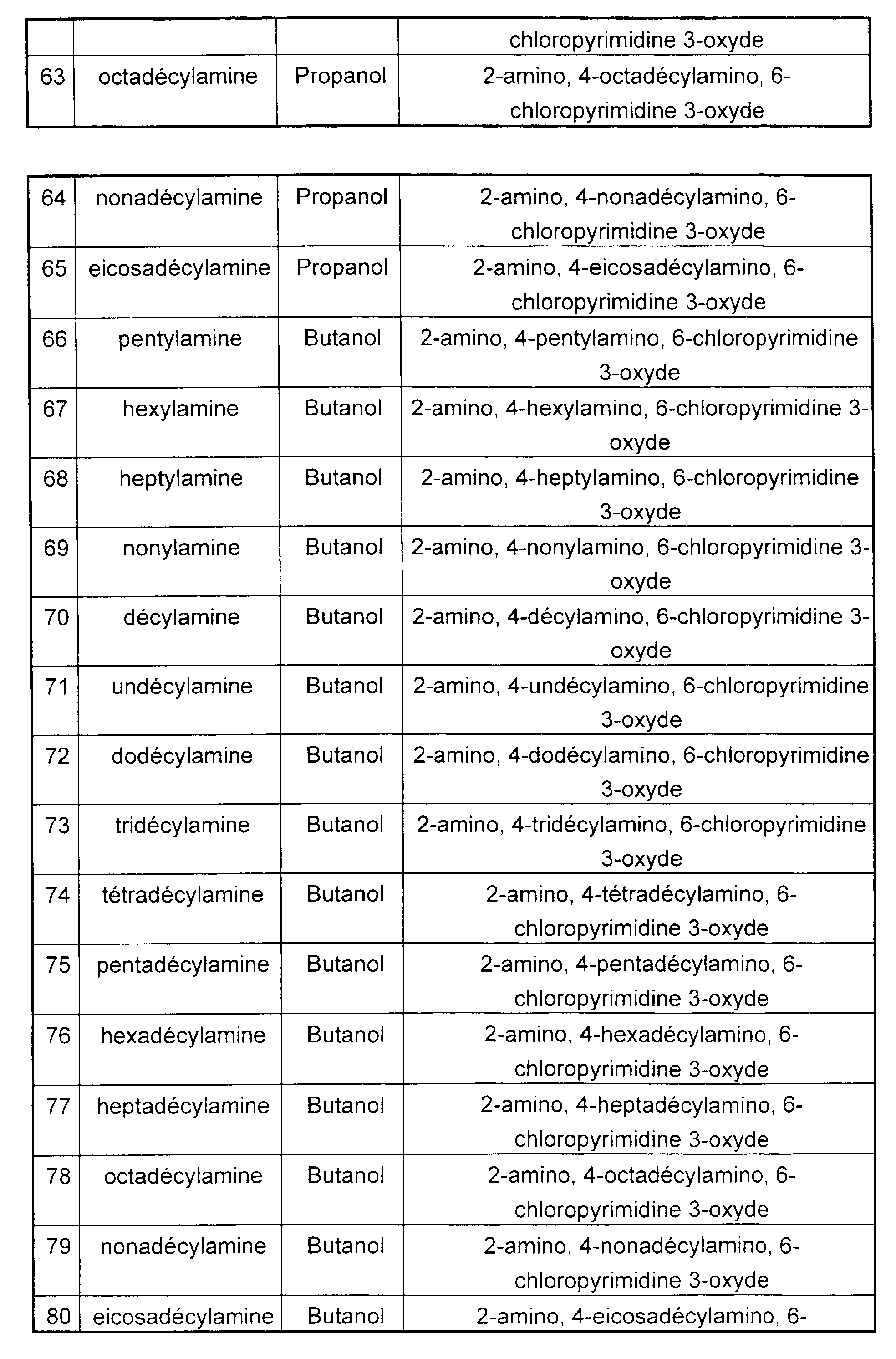

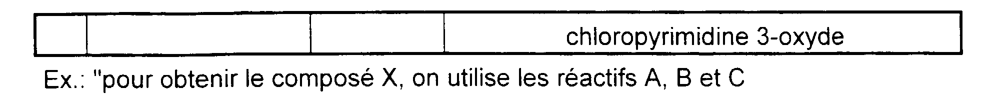

### Exemple 81 :

Mesure du pouvoir activateur de la prostaglandine-endoperoxyde synthase (PGHS-1).

### Principes généraux de mesure :

On mesure la quantité d'oxygène nécessaire à l'oxydation de l'acide arachidonique par l'activité cyclooxygénase de la prostaglandine-endoperoxyde synthase, en présence ou en absence du dérivé à tester.

Les mesures de la consommation d'oxygène sont effectuées avec une électrode de Clark raccordée à un oxymétre YSI 5300 de marque Yellow Spring Instruments.
Ces mesures sont effectuées en chambre ouverte sous agitation constante à la température de 37°C.
Si l'on utilise un enregistreur graphique, la mesure de la consommation en oxygène se matérialise sous la forme d'une courbe, dont la pente maximale permet de déduire la vitesse initiale de la réaction, et à partir de laquelle il est possible de calculer la durée pendant laquelle la vitesse initiale de la réaction est maintenue.

La courbe ainsi obtenue, en l'absence de toute substance autre que les ingrédients nécessaires à la réaction enzymatique, donne l'activité de base de l'enzyme. On peut déterminer dans ces conditions la vitesse initiale et la durée pendant laquelle cette vitesse est maintenue, dans une réaction ne comportant que l'enzyme et son substrat.
Ces données serviront de référence à l'étude des dérivés à tester.

La mesure de l'activité des dérivés à tester s'effectue dans les mêmes conditions, en ajoutant au milieu réactionnel le dérivé à tester. Ce sont la variation de la pente et la variation de la durée pendant laquelle la vitesse maximale est maintenue qui permettent d'évaluer l'activité du dérivé à tester vis à vis de la cyclooxygénase.

### Préparation aux mesures :

On prépare une solution de TRIS 0,1 M et d'EDTA 5 mM à pH = 8,00 (solution TE).
Les mesures s'effectuent dans une solution tamponnée (Tampon TEA) composée de 9 volumes de solution TE et 1 volume d'alcool à 20%.

Le substrat est préparé sous la forme d'une solution mère d'arachidonate de potassium selon le protocole du fournisseur (Interchim, France).
La solution ainsi obtenue titre à 46 mM en acide arachidonique. Elle peut être stockée à 4°C pour 24 heures.

L'enzyme utilisée est de la prostaglandine-endoperoxyde synthase (PGHS-1), isolée de glandes séminales de mouton, vendue par la société Cayman Chemical sous la référence 60100.

Les dérivés à tester sont préparés sous forme de solution mère titrant à 5 mM dans un mélange eau/alcool (80/20) et testés avec un même lot d'enzyme.

### Mesures :

### Activités de base de l'enzyme :

A t = 0, on introduit dans la chambre de mesure 380 µl de tampon TEA préchauffé à 37°C, qu'on laisse s'équilibrer au moins une minute.
A t = 1, on introduit 300 unités d'enzyme (PGHS).
L'enregistrement est déclenché, et on laisse l'ensemble se stabiliser de nouveau pendant une minute. L'enregistrement obtenu donne le niveau de base de la réaction.
Au bout d'une minute supplémentaire, on introduit 10 µl de substrat et l'on enregistre la consommation en oxygène pendant 2 à 3 minutes.

On détermine ainsi la vitesse initiale de la réaction et la durée pendant laquelle cette vitesse est maintenue. Ces données serviront de référence aux mesures de l'activité des dérivés à tester.

### Activités des dérivés à tester :

Les conditions d'expérimentation sont identiques aux précédentes, si ce n'est que le tampon TEA préchauffé à 37°C est remplacé par un tampon identique contenant le dérivé à tester à la concentration de 0,5 mM.

### Résultats :

Ces résultats sont exprimés en % par rapport aux valeurs obtenues avec le témoin.

| Dérivés | Activation |
|---|---|
| Témoin | +0% |
| Composé de l'exemple 1 | +37% |
| Composé de l'exemple 2 | +11% |
| Composé de l'exemple 3 | +14% |
| Composé de l'exemple 17 | +8% |
| Indométhacine¹ | -100% |

| | |
|---|---|
| ¹: le 1-(4-chlorobenzoyl)-5-methoxy-2 methyl-1H-indole 3 acide acétique ou indométhacine est un inhibiteur connu de l'activité cyclooxygénase. (H.P Rang / M.M.Dale Pharmacology second édition 1991. Churchill Livingstone édition). Dans les conditions de l'expérience, on retrouve bien cet effet inhibiteur. | |

### Exemple 82 :

Exemples de compositions contenant au moins un 2-amino, 4-alkylamino pyrimidine 3-oxyde de l'invention.
Ces compositions sont obtenues par les techniques habituelles couramment utilisées en cosmétique ou en pharmacie.

### Lotion antichute :

- Composé de l'exemple 2 0.5 g
- Propylène glycol 10,0 g
- Alcool isopropylique qsp 100,0 g

On applique 1 ml de cette lotion sur le cuir chevelu, à la fréquence de une à deux fois par jour.

### Gel niosomé :

- Chimexane NS① 1,8 g
- Stéaroylglutamate monosodique 0,2 g
- Composé de l'exemple 17 2,0 g
- Carbomer 0,2 g
- Triéthanolamine qs pH = 7
- Conservateurs qs
- Parfums qs
- Eau déminéralisée qsp 100,0 g

On applique ce gel sur le cuir chevelu, une à deux fois par jour.

### Lotion antichute :

- Composé de l'exemple 17 3,0 g
- Propylène glycol 30,0 g
- Alcool éthylique 40,5 g
- Eau qsp 100,0 g

On applique cette lotion sur le cuir chevelu, une à deux fois par jour, à raison d'1 ml par application.

### Lotion antichute épaissie :

- Composé de l'exemple 2 1,0 g
- Kawaïne 2,0 g
- Hydroxypropylcellulose vendue par la société Hercules sous la dénomination Klucel G 3,5 g
- Alcool éthylique qsp 100,0 g

On applique cette lotion épaissie sur le cuir chevelu, une à deux fois par jour, à raison d'1 ml par application.

### Lotion niosomée :

- Chimexane NL① 0,475 g
- Cholestérol 0,475 g
- Stéaroylglutamate monosodique 0,05 g
- Composé de l'exemple 1 1,0 g
- Conservateurs qs
- Colorants qs
- Parfum qs
- Eau déminéralisée qsp 100,0 g

On applique cette lotion sur le cuir chevelu, une à deux fois par jour, à raison d'1 ml par application.

### lotion antichute :

- Composé de l'exemple 3 1,0 g
- Monométhyléther de propylèneglycol vendu sous la dénomination Dowanol PM par la société Dow Chemical 20,0 g
- Hydroxypropylcellulose vendue par la société Herculès sous la dénomination Klucel G 3,0 g
- Alcool éthylique 40,0 g
- Eau qsp 100,0 g

On applique cette lotion épaissie sur le cuir chevelu, une à deux fois par jour, à raison d'1 ml par application.

## Revendications

1. Composé chimique de la famille des 2-amino, 4-alkylamino pyrimidine 3-oxydes répondant à la formule générale (I) : 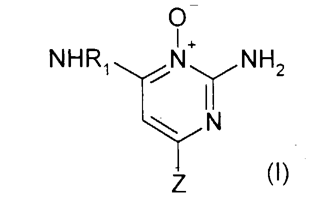 dans laquelle
R₁ représente un groupement alkyle ayant de 5 à 20 atomes de carbone,
et Z représente soit un atome d'hydrogène, soit le radical -OR₂, dans lequel R₂ représente un groupement alkyle ayant de 1 à 12 atomes de carbone,
ainsi que ses formes acylées ou ses sels d'addition d'acides.

2. Composé selon la revendication précédente, caractérisé par le fait que R₁ est un groupement alkyle ayant de 6 à 12 atomes de carbone.

3. Composé selon l'une quelconque des revendications précédentes, caractérisé par le fait que R₂ est un groupement alkyle ayant de 1 à 12 atomes de carbone.

4. Composé selon l'une quelconque des revendications précédentes, caractérisé par le fait est choisi parmi le :
2-amino, 4-pentylamino pyrimidine 3-oxyde,
2-amino, 4-hexylamino pyrimidine 3-oxyde,
2-amino, 4-heptylamino pyrimidine 3-oxyde,
2-amino, 4-octylamino pyrimidine 3-oxyde,
2-amino, 4-nonylamino pyrimidine 3-oxyde,
2-amino, 4-décylamino pyrimidine 3-oxyde,
2-amino, 4-undécylamino pyrimidine 3-oxyde,
2-amino, 4-dodécylamino pyrimidine 3-oxyde,
2-amino, 4-tridécylamino pyrimidine 3-oxyde,
2-amino, 4-tétradécylamino pyrimidine 3-oxyde,
2-amino, 4-pentadécylamino pyrimidine 3-oxyde,
2-amino, 4-hexadécylamino pyrimidine 3-oxyde,
2-amino, 4-heptadécylamino pyrimidine 3-oxyde,
2-amino, 4-octatadécylamino pyrimidine 3-oxyde,
2-amino, 4-nonadécylamino pyrimidine 3-oxyde,
2-amino, 4-eicosadécylamino pyrimidine 3-oxyde,
2-amino, 4-pentylamino, 6-méthoxy pyrimidine 3-oxyde,
2-amino, 4-hexylamino, 6-méthoxy pyrimidine 3-oxyde,
2-amino, 4-heptylamino, 6-méthoxy pyrimidine 3-oxyde,
2-amino, 4-octylamino, 6-méthoxy pyrimidine 3-oxyde,
2-amino, 4-nonylamino, 6-méthoxy pyrimidine 3-oxyde,
2-amino, 4-décylamino, 6-méthoxy pyrimidine 3-oxyde,
2-amino, 4-undécylamino, 6-méthoxy pyrimidine 3-oxyde,
2-amino, 4-dodécylamino, 6-méthoxy pyrimidine 3-oxyde,
2-amino, 4-tridécylamino, 6-méthoxy pyrimidine 3-oxyde,
2-amino, 4-tétradécylamino, 6-méthoxy pyrimidine 3-oxyde,
2-amino, 4-pentadécylamino, 6-méthoxy pyrimidine 3-oxyde,
2-amino, 4-hexadécylamino, 6-méthoxy pyrimidine 3-oxyde,
2-amino, 4-heptadécylamino, 6-méthoxy pyrimidine 3-oxyde,
2-amino, 4-octatadécylamino, 6-méthoxy pyrimidine 3-oxyde,
2-amino, 4-nonadécylamino, 6-méthoxy pyrimidine 3-oxyde,
2-amino, 4-eicosadécylamino, 6-méthoxy pyrimidine 3-oxyde,
2-amino, 4-pentylamino, 6-éthoxy pyrimidine 3-oxyde,
2-amino, 4-hexylamino, 6-éthoxy pyrimidine 3-oxyde,
2-amino, 4-heptylamino, 6-éthoxy pyrimidine 3-oxyde,
2-amino, 4-octylamino, 6-éthoxy pyrimidine 3-oxyde,
2-amino, 4-nonylamino, 6-éthoxy pyrimidine 3-oxyde,
2-amino, 4-décylamino, 6-éthoxy pyrimidine 3-oxyde,
2-amino, 4-undécylamino, 6-éthoxy pyrimidine 3-oxyde,
2-amino, 4-dodécylamino, 6-éthoxy pyrimidine 3-oxyde,
2-amino, 4-tridécylamino, 6-éthoxy pyrimidine 3-oxyde,
2-amino, 4-tétradécylamino, 6-éthoxy pyrimidine 3-oxyde,
2-amino, 4-pentadécylamino, 6-éthoxy pyrimidine 3-oxyde,
2-amino, 4-hexadécylamino, 6-éthoxy pyrimidine 3-oxyde,
2-amino, 4-heptadécylamino, 6-éthoxy pyrimidine 3-oxyde,
2-amino, 4-octatadécylamino, 6-éthoxy pyrimidine 3-oxyde,
2-amino, 4-nonadécylamino, 6-éthoxy pyrimidine 3-oxyde,
2-amino, 4-eicosadécylamino, 6-éthoxy pyrimidine 3-oxyde,
2-amino, 4-pentylamino, 6-propyloxy pyrimidine 3-oxyde,
2-amino, 4-hexylamino, 6-propyloxy pyrimidine 3-oxyde,
2-amino, 4-heptylamino, 6-propyloxy pyrimidine 3-oxyde,
2-amino, 4-octylamino, 6-propyloxy pyrimidine 3-oxyde,
2-amino, 4-nonylamino, 6-propyloxy pyrimidine 3-oxyde,
2-amino, 4-décylamino, 6-propyloxy pyrimidine 3-oxyde,
2-amino, 4-undécylamino, 6-propyloxy pyrimidine 3-oxyde,
2-amino, 4-dodécylamino, 6-propyloxy pyrimidine 3-oxyde,
2-amino, 4-tridécylamino, 6-propyloxy pyrimidine 3-oxyde,
2-amino, 4-tétradécylamino, 6-propyloxy pyrimidine 3-oxyde,
2-amino, 4-pentadécylamino, 6-propyloxy pyrimidine 3-oxyde,
2-amino, 4-hexadécylamino, 6-propyloxy pyrimidine 3-oxyde,
2-amino, 4-heptadécylamino, 6-propyloxy pyrimidine 3-oxyde,
2-amino, 4-octatadécylamino, 6-propyloxy pyrimidine 3-oxyde,
2-amino, 4-nonadécylamino, 6-propyloxy pyrimidine 3-oxyde,
2-amino, 4-eicosadécylamino, 6-propyloxy pyrimidine 3-oxyde,
2-amino, 4-pentylamino, 6-butyloxy pyrimidine 3-oxyde,
2-amino, 4-hexylamino, 6-butyloxy pyrimidine 3-oxyde,
2-amino, 4-heptylamino, 6-butyloxy pyrimidine 3-oxyde,
2-amino, 4-octylamino, 6-butyloxy pyrimidine 3-oxyde,
2-amino, 4-nonylamino, 6-butyloxy pyrimidine 3-oxyde,
2-amino, 4-décylamino, 6-butyloxy pyrimidine 3-oxyde,
2-amino, 4-undécylamino, 6-butyloxy pyrimidine 3-oxyde,
2-amino, 4-dodécylamino, 6-butyloxy pyrimidine 3-oxyde,
2-amino, 4-tridécylamino, 6-butyloxy pyrimidine 3-oxyde,
2-amino, 4-tétradécylamino, 6-butyloxy pyrimidine 3-oxyde,
2-amino, 4-pentadécylamino, 6-butyloxy pyrimidine 3-oxyde,
2-amino, 4-hexadécylamino, 6-butyloxy pyrimidine 3-oxyde,
2-amino, 4-heptadécylamino, 6-butyloxy pyrimidine 3-oxyde,
2-amino, 4-octatadécylamino, 6-butyloxy pyrimidine 3-oxyde,
2-amino, 4-nonadécylamino, 6-butyloxy pyrimidine 3-oxyde,
2-amino, 4-eicosadécylamino, 6-butyloxy pyrimidine 3-oxyde,
et leurs isomères ramifiés .

5. Composé selon la revendication précédente, caractérisée par le fait qu'il est choisi parmi le
2-amino, 4-hexylamino pyrimidine 3-oxyde,
2-amino, 4-octylamino pyrimidine 3-oxyde,
2-amino, 4-dodécylamino pyrimidine 3-oxyde,
2-amino, 4-octylamino, 6-butyloxy pyrimidine 3-oxyde,

6. Composé selon la revendication précédente, caractérisée par le fait qu'il est choisi parmi le
2-amino, 4-dodécylamino pyrimidine 3-oxyde,
2-amino, 4-octylamino, 6-butyloxy pyrimidine 3-oxyde,

7. Procédé de préparation des 2-amino, 4-alkylamino pyrimidine 3-oxydes de formule (I) tels que définis selon l'une quelconque des revendications 1 à 6, caractérisé par le fait que l'on fait réagir dans un solvant comme l'éthanol une amine aliphatique et de la 2-amino, 4,6-dichloropyrimidine, que le composé ainsi obtenu après purification est ensuite mis à réagir dans un solvant comme l'isopropanol avec un complexe Urée/H₂O₂ et de l'anhydride phtalique et qu'après purification, le composé obtenu est mis à réagir dans un solvant comme l'éthanol absolu en présence de potasse, de palladium sur charbon sous haute pression d'hydrogène.

8. Procédé de préparation des 2-amino, 4-alkylamino, 6-alkyloxy pyrimidine 3-oxydes de formule (I) tels que définis selon l'une quelconque des revendications 1 à 6, caractérisé par le fait que l'on fait réagir dans un solvant comme l'éthanol une amine aliphatique et de la 2-amino, 4,6-dichloropyrimidine, que le composé ainsi obtenu après purification est ensuite mis à réagir en présence d'isopropanol avec un complexe Urée/H₂O₂ et de l'anhydride phtalique et qu'après purification, le composé obtenu est mis à réagir avec un excès d'alkanolate de sodium pour obtenir le 2-amino, 4-alkylamino, 6-alkyloxy pyrimidine 3-oxyde correspondant.

9. Composition comprenant au moins un composé de formule (I) tel que défini selon l'une quelconque des revendications 1 à 6.

10. Composition selon la revendication 9, caractérisée par le fait qu'elle comprend au moins un composé de formule (I) en une quantité représentant de 0.001% à 10% du poids total de la composition et préférentiellement en une quantité représentant de 0.01% à 2% du poids total de la composition.

11. Composition selon l'une quelconque des revendications 9 ou 10, caractérisée par le fait qu'elle est destinée à un usage cosmétique ou pharmaceutique.

12. Composition selon l'une quelconque des revendications 9 à 11, caractérisée par le fait qu'elle comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale, marine ou bactérienne.

13. Composition selon la revendication 12, caractérisée par le fait l'antifongique est choisi parmi les imidazoles.

14. Composition selon la revendication 13, caractérisée par le fait l'antifongique est le kétoconazole.

15. Composition selon l'une quelconque des revendications 9 à 14, caractérisée par le fait qu'elle est destinée à induire et/ou stimuler la croissance des cheveux et/ou freiner leur chute.

16. Utilisation dans une composition ou pour la préparation d'une composition, d'une quantité efficace d'au moins un composé répondant à la formule générale (I) tel que défini selon l'une quelconque des revendications 1 à 7, ce composé ou cette composition étant destiné à induire et/ou stimuler la croissance des cheveux et/ou freiner leur chute.

17. Procédé de traitement cosmétique des cheveux et/ou du cuir chevelu, caractérisé par le fait qu'il consiste à appliquer sur les cheveux et/ou le cuir chevelu et/ou sur la peau, une composition cosmétique telle que définie dans l'une quelconque des revendications 9 à 15, à laisser celle-ci en contact avec les cheveux et/ou le cuir chevelu et/ou la peau, et éventuellement à rincer.
